# EUROPEAN PATENT APPLICATION

(11) **EP 4 356 942 A1**
(43) Date of publication of application: **24.04.2024**
(21) Application number: 22824679.9
(22) Date of filing: 19.04.2022
(51) Int. Cl.: A61M 5/14, A61M 5/145, A61M 25/09

(54) **RESIN-COATED WIRE AND LIQUID MEDICINE INJECTION DEVICE**

(30) Priority: 16.06.2021 JP 2021099904
(71) Applicant: Asahi Intecc Co., Ltd., Seto-shi, Aichi 489-0071 (JP)
(72) Inventor: SHIBAKAWA, Risa, Seto-shi, Aichi 489-0071 (JP); NAMIMA, Satoshi, Seto-shi, Aichi 489-0071 (JP); TAKEMOTO, Hirokatsu, Seto-shi, Aichi 489-0071 (JP)
(74) Representative: Prinz & Partner mbB
(86) International application number: PCT/JP2022/018177
(87) International publication number: WO 2022/264684

(57) **Abstract**

Resin-coated wire is capable of moving forward inside a drug solution-containing lumen of a catheter containing a drug solution, and thereby pushing out the drug solution from a distal end of the catheter. The resin-coated wire includes a wire, and a coated portion made of resin coating the wire. The coated portion has a distal end coated portion and a proximal end coated portion. The distal end coated portion is formed of a first resin material and coats a first part, which is a distal end portion of the wire. The proximal end coated portion is formed of a second resin material and coats a second part adjacent to a proximal end side of the first part of the wire. The first resin material has a higher affinity for water than the second resin material.

## Description

### TECHNICAL FIELD

The technology disclosed herein relates to a resin-coated wire and a drug solution injection device.

### BACKGROUND ART

As a drug solution injection device for injecting a drug solution into the body of a patient, a drug solution injection device is known which includes an outer tube having a hollow portion into which an inner tube with a puncture needle at a distal end is inserted to inject a drug solution from the puncture needle into the body of a patient via a hollow portion of the inner tube (see, e.g., Patent Literature 1).

### CITATION LIST

### Patent Literature

Patent Literature 1: Japanese Patent Application Publication No. 2013-172842

### SUMMARY OF INVENTION

### TECHNICAL PROBLEM

Another type of drug solution injection device is a drug solution injection device capable of moving wire forward for a predetermined distance inside a drug solution-containing lumen of a catheter containing a drug solution, and thereby discharging a predetermined amount of the drug solution from a distal end of the catheter. With such a type of drug solution injection device it is possible to accurately control the discharge amount of the drug solution, as well as to reduce loss of the drug solution.

However, a drug solution injection device having this configuration has a problem in that when a bubble is mixed in a drug solution contained in a drug solution-containing lumen of a catheter, the bubble may be administered into an affected area of a patient together with the drug solution.

The technology disclosed herein provides a solution for the problem described above.

### SOLUTION TO PROBLEM

The technology disclosed herein can be achieved e.g., in the following forms.
(1) The resin-coated wire disclosed herein is capable of moving forward inside a drug solution-containing lumen of a catheter containing a drug solution, and thereby pushing out the drug solution from a distal end of the catheter. The resin-coated wire includes a wire, and a coated portion made of resin coating the wire. The coated portion includes a distal end coated portion and a proximal end coated portion. The distal end coated portion is formed of a first resin material and coats a first part, which is a distal end portion of the wire. The proximal end coated portion is formed of a second resin material and coats a second part adjacent to a proximal end side of the first part of the wire. The first resin material has a higher affinity for water than the second resin material.
   In the resin-coated wire, a resin material for forming a distal end coated portion has a higher affinity for water than a resin material for forming a proximal end coated portion. In other words, a bubble in water (in the drug solution) easily attaches to and spreads on a surface of the proximal end coated portion, while the bubble poorly spreads and insufficiently attaches onto a surface of the distal end coated portion. Hence, even when a bubble is mixed in the drug solution contained in the drug solution-containing lumen of the catheter, the bubble can be prevented from flowing to the distal end by moving the resin-coated wire forward and causing the bubble to move from a position of the distal end coated portion to a position of the proximal end coated portion and attach to the proximal end coated portion. Accordingly, the resin-coated wire can reduce the risk of administering a bubble together with a drug solution into an affected area, in pushing out the drug solution from the distal end of the catheter using the resin-coated wire.
(2) The resin-coated wire may have a configuration where the wire is a rope wire formed by spirally winding a plurality of fine wires. The resin-coated wire can increase flexibility of the resin-coated wire, and the resin-coated wire can be easily moved forward inside a drug solution-containing lumen of a catheter even at when the catheter is bent, and the maneuverability of the resin-coated wire and accuracy of drug solution injection by the resin-coated wire can be improved.
(3) The resin-coated wire may have a configuration where each of the plurality of fine wires is a stranded wire formed by spirally winding a plurality of strands. According to this resin-coated wire, the flexibility of the resin-coated wire can be effectively improved, and even if the catheter is bent, the resin-coated wire can be more easily moved forward inside the drug solution-containing lumen of the catheter, and the maneuverability of the resin-coated wire and accuracy of drug solution injection by the resin-coated wire can be effectively improved.
(4) The resin-coated wire may have a configuration where the first resin material permeates a distal end part of the second part of the wire. According to this resin-coated wire, loss of a drug solution due to e.g., infiltration of a drug solution from a boundary between the proximal end coated portion and the distal end coated portion to an inside of the wire can be effectively reduced. The resin-coated wire can also avoid a sharp change of rigidity of the wire (and thus of rigidity of the resin-coated wire) from the distal end to the proximal end of the wire, and improve maneuverability of the resin-coated wire and accuracy of drug solution injection by the resin-coated wire.
(5) The resin-coated wire may have a configuration where a most distal end portion of the resin-coated wire has an approximate hemisphere shape forming a projection toward a distal end. According to this resin-coated wire, even if the catheter is bent, the resin-coated wire can be more easily moved forward inside the drug solution-containing lumen of the catheter, and maneuverability of the resin-coated wire and accuracy of drug solution injection by the resin-coated wire can be effectively improved.
(6) The resin-coated wire may have a configuration where the resin-coated wire includes a most distal end portion, an intermediate portion adjacent to a proximal end side of the most distal end portion and having a taper shape with an outer diameter reduced toward a distal end, and a proximal end portion adjacent to a proximal end side of the intermediate portion and having an approximately constant outer diameter. Owing to having the tapered intermediate portion, according to this resin-coated wire, even if the catheter is bent, the resin-coated wire can be more easily moved forward inside the drug solution-containing lumen of the catheter, and maneuverability of the resin-coated wire and accuracy of drug solution injection by the resin-coated wire can be effectively improved.
(7) The resin-coated wire may have a configuration where a part formed of the first part of the wire has higher rigidity than that of a part formed of the second part of the wire. According to this resin-coated wire, when the resin-coated wire is moved forward inside the drug-solution containing lumen of the catheter, the risk of deformation or backward movement of a vicinity of the distal end of the resin-coated wire due to a pressure from the drug solution can be reduced, , and maneuverability of the resin-coated wire and accuracy of drug solution injection by the resin-coated wire can be improved.
(8) A drug solution injection device disclosed herein includes the resin-coated wire described above and a catheter. With this drug solution injection device, the maneuverability, accuracy of drug solution injection, etc. of a drug solution injection device can be improved.

The technology disclosed herein can be implemented in various modes, such as a resin-coated wire, a drug solution injection device including a resin-coated wire, a system including a drug solution injection device, and a method of producing such a device or system.

### BRIEF DESCRIPTION OF DRAWINGS

FIG. 1 is an explanatory view schematically illustrating a configuration of a drug solution injection device 100 according to an embodiment.
FIG. 2 is an explanatory view illustrating a detailed configuration of a resin-coated wire 20.
FIG. 3 is an explanatory view illustrating a detailed configuration of the resin-coated wire 20.
FIG. 4 is an explanatory view illustrating a detailed configuration of the resin-coated wire 20.

### EMBODIMENTS OF THE INVENTION

### A. Embodiment:

### A-1. Configuration of Drug Solution Injection Device 100:

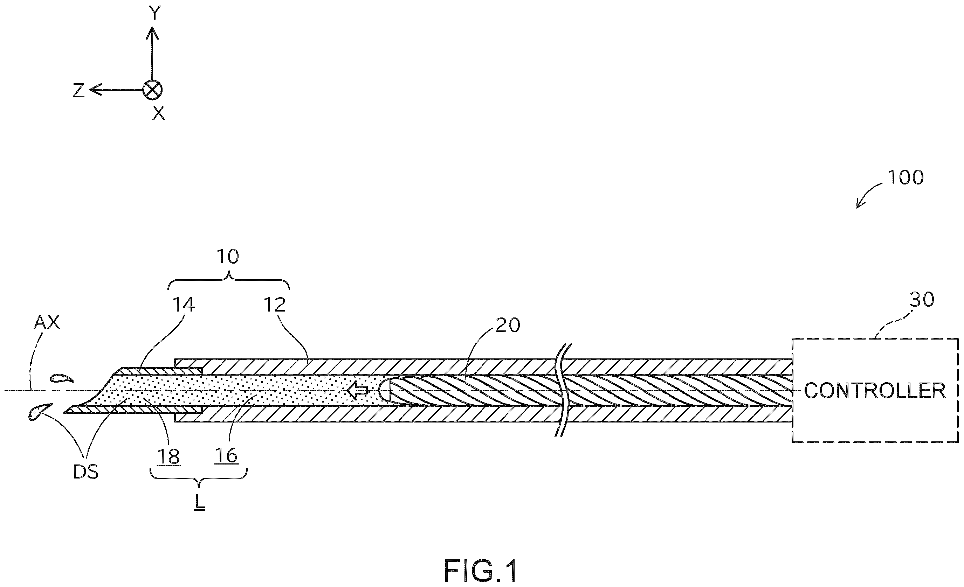

FIG. 1 is an explanatory view schematically illustrating a configuration of a drug solution injection device 100 according to an embodiment. FIG. 1 depicts a configuration of a longitudinal section (Y-Z section) of the drug solution injection device 100. In FIG. 1, the positive direction on the Z-axis is toward a distal end (farther side) to be inserted into the body, and the negative direction on the Z-axis is toward a proximal end (near side) to be manipulated by a professional such as a physician. FIG. 1 omits or schematically depicts a configuration of a part of the drug solution injection device 100. These points are also the same in the drawings shown below. Herein, in the drug solution injection device 100 and the component member thereof, the terminal of a distal part is referred to as "distal end", the distal end and the vicinity thereof is referred to as "distal end portion", the terminal of a proximal part is referred to as "proximal end", and the proximal end and the vicinity thereof is referred to as "proximal end portion".

The drug solution injection device 100 is a medical device used for injecting a drug solution into the body of a patient by employing a catheter capable of being inserted into a living body lumen. A living body lumen herein encompasses a variety of lumens such as a blood vessel, a lymphatic vessel, a biliary tract, a urinary tract, a respiratory tract, a gastrointestinal tract, a secretory gland, and a genital tract. The drug solution injection device 100 includes a needle catheter 10, a resin-coated wire 20, and a controller 30.

The needle catheter 10 is a member used for being inserted into a living body lumen of a patient via e.g., a channel of an endoscope, delivering a drug solution to a desired site in the body of the patient, and injecting the drug solution into the site. The needle catheter 10 includes a catheter shaft 12 and a needle portion 14. The catheter shaft 12 is a long member extending along a center axis AX. In more detail, the catheter shaft 12 is an approximately cylindrical member that includes an opening formed at each of a distal end and a proximal end, and an inner cavity 16 formed inside and connecting the two openings. FIG. 1 illustrates the center axis AX of the catheter shaft 12 linearly arranged parallel to the Z-axis, and the catheter shaft 12 has a flexibility to the extent that it can be bend. The needle portion 14 is a hollow member attached to a distal end portion of the catheter shaft 12 and including an inner cavity 18 formed so as to communicate with the inner cavity 16 of the catheter shaft 12. The inner cavity 16 of the catheter shaft 12 and the inner cavity 18 of the needle portion 14 function as a drug solution-containing lumen L containing a drug solution DS. An exemplary transverse sectional shape of the drug solution-containing lumen L is an approximate round shape having an approximately constant inner diameter. At a distal end of the needle portion 14, a needle tip is formed for puncturing an injection site of a drug solution. The needle catheter 10 is an exemplary catheter.

The catheter shaft 12 preferably has an antithrombotic property, flexibility, and biocompatibility, and can be formed of e.g., a resin material or a metal material. Examples of a resin material available for forming the catheter shaft 12 include polyamide resins, polyolefin resins, polyester resins, polyurethane resins, silicone resins, and fluororesins. Examples of a metal material available for forming the catheter shaft 12 include stainless steel such as SUS304, Ni-Ti alloy, and cobalt-chromium alloy. In addition, a coil body, a braided body or the like may be embedded in a thick part of the catheter shaft 12 for improving at least a part of flexibility, torquability, push-in ability, kink resistance, blood vessel conformability, migration in a lesion, and maneuverability. The catheter shaft 12 may also be formed of a plurality of layers formed of the same or different materials. The needle portion 14 preferably has an antithrombotic property and biocompatibility, and can be formed of e.g., stainless steel such as SUS304, Ni-Ti alloy, or a metal material such as cobalt-chromium alloy.

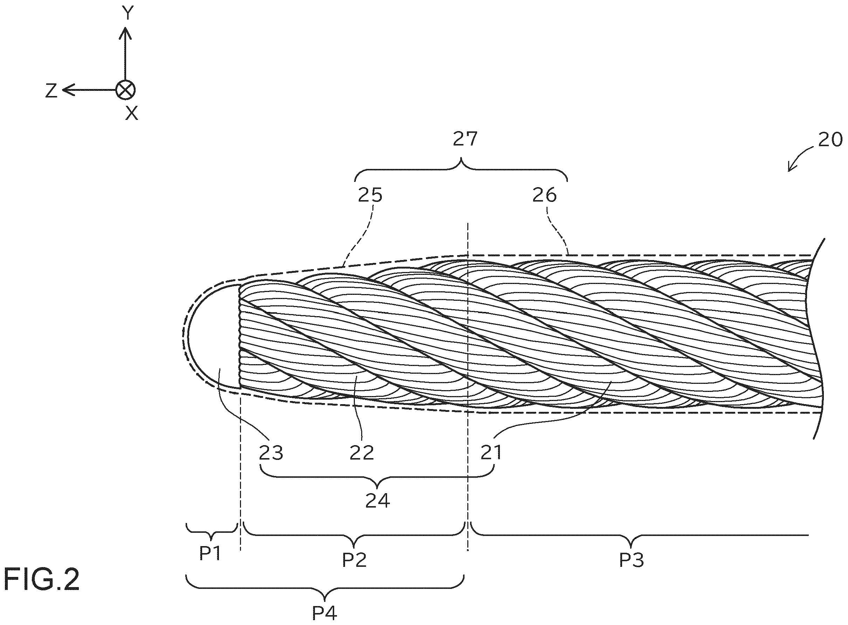

The resin-coated wire 20 is a member for being inserted from a proximal end of the needle catheter 10 into the drug solution-containing lumen L, pushing out the drug solution DS contained in the drug solution-containing lumen L toward a distal end by moving forward inside the drug solution-containing lumen L, and thereby discharging the drug solution DS from a distal end of the needle portion 14. The resin-coated wire 20 is a long member extending along the center axis AX. FIG. 1 illustrates the center axis AX of the resin-coated wire 20 linearly arranged parallel to the Z-axis, and the resin-coated wire 20 has flexibility to an extent that it can be bend. The outer diameter of the resin-coated wire 20 (in more detail, the outer diameter of the proximal end portion P3 described later (FIG. 2) of the resin-coated wire 20 is approximately the same (clearance: 100 µm or less) as the inner diameter of the drug solution-containing lumen L formed in the needle catheter 10. A configuration of the resin-coated wire 20 is described later in detail.

The controller 30 is a device including e.g., a gear mechanism, a roller, and a discharge operation part and capable of moving forward, for a predetermined distance, the resin-coated wire 20 inserted into the drug solution-containing lumen L of the needle catheter 10. When the controller 30 delivers the resin-coated wire 20 for a predetermined distance toward the distal end, the drug solution DS contained in the drug solution-containing lumen L of the needle catheter 10 is pushed toward the distal end by the resin-coated wire 20, and an amount of the drug solution DS equivalent to the volume of the resin-coated wire 20 thus forwarded is discharged from the distal end of the needle portion 14. Such an operation is repeated predetermined times, thereby allowing injection of a predetermined amount of the drug solution DS into the body of a patient.

### A-2. Detailed Configuration of Resin-coated Wire 20:

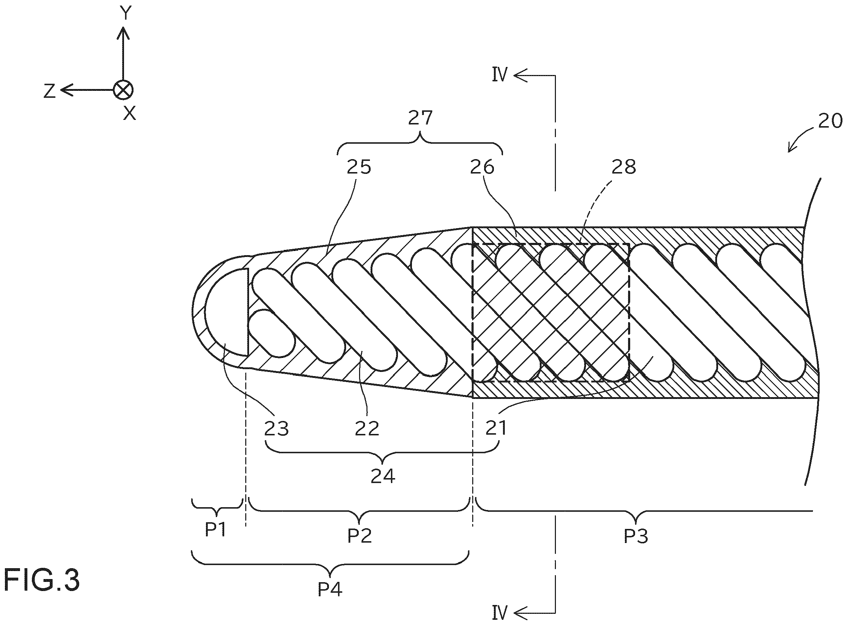

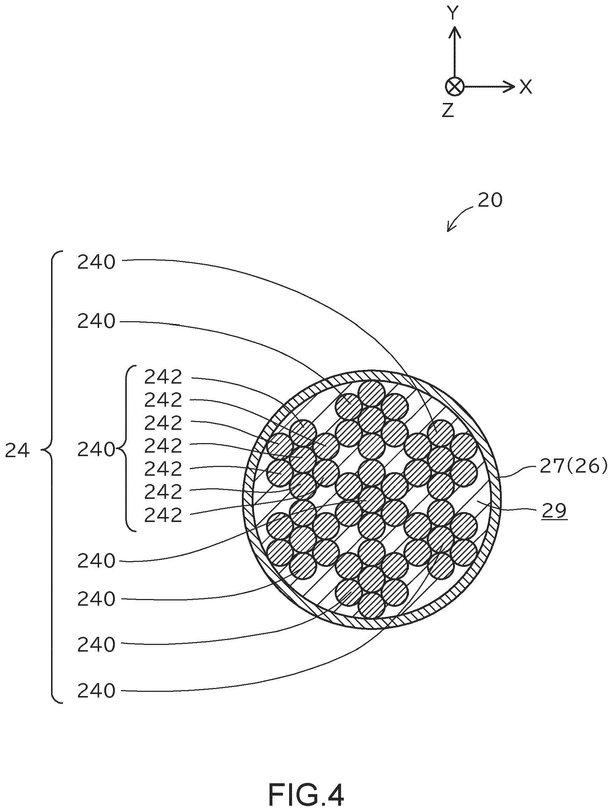

Next, description will be made for a detailed configuration of the resin-coated wire 20. FIGs. 2-4 are explanatory views illustrating a detailed configuration of the resin-coated wire 20. FIG. 2 depicts a configuration of an external appearance of a part close to the distal end in the resin-coated wire 20. FIG. 3 schematically depicts a configuration of a longitudinal section (Y-Z section) of a part close to the distal end in the resin-coated wire 20. FIG. 4 depicts a configuration of a transverse section (X-Y section) of the resin-coated wire 20 taken along the line IV-IV in FIG. 3.

As depicted in FIGs. 2-4, the resin-coated wire 20 has a wire 24, and a coated portion 27 made of a resin and coating the wire 24. In the embodiment, the coated portion 27 is approximately transparent, and FIG. 2 thus indicates the coated portion 27 by a dashed line.

The wire 24 is a long member extending along the center axis AX. As depicted in FIG. 4, in the embodiment, the wire 24 is a rope wire formed by spirally winding a plurality (seven) of the fine wires 240. In the embodiment, each of the plurality of the fine wires 240 forming the wire 24 is a stranded wire formed by spirally winding a plurality (seven) of strands 242. The wire 24 preferably has an antithrombotic property, flexibility, and biocompatibility, and can be formed of e.g., a metal material. Examples of a metal material available for forming the wire 24 include stainless steel such as SUS304, Ni-Ti alloy, or cobalt-chromium alloy.

As depicted in FIG. 2 and FIG. 3, the wire 24 has a straight portion 21, a welded portion 23 at the most distal end of the wire 24, and a tapered portion 22 connecting the straight portion 21 to the welded portion 23. The straight portion 21 is a part having an approximately constant outer diameter. The outer diameter of the straight portion 21 is e.g., about 0.5 mm to 2.0 mm. In the embodiment, the straight portion 21 continues to a proximal end of the wire 24. The welded portion 23 is a part including joining among the fine wires 240 and/or among the strands 242, all of which are included in the wire 24, by welding using a predetermined method of welding (e.g., YAG laser welding), and a part having an approximate hemisphere shape forming a projection toward a distal end. The tapered portion 22 is a part having a taper shape with an outer diameter reduced toward the distal end. The outer diameter of a proximal end of the tapered portion 22 is approximately the same as the outer diameter of the straight portion 21, and the outer diameter of a distal end of the tapered portion 22 is approximately the same as the outer diameter of a proximal end of the welded portion 23. An example of the shape of a transverse section at each site of the wire 24 is approximately circular. The welded portion 23 and the tapered portion 22 of the wire 24 is an exemplary first part, and the straight portion 21 of the wire 24 is an exemplary second part.

In the following description, the resin-coated wire 20 is virtually separated into several parts along the center axis AX, and a part including the welded portion 23 of the wire 24 is referred to as a most distal end portion P1, a part including the tapered portion 22 of the wire 24 is referred to as an intermediate portion P2, and a part including the straight portion 21 of the wire 24 is referred to as a proximal end portion P3. The most distal end portion P1 and the intermediate portion P2 are collectively referred to as a distal end portion P4.

The coated portion 27 is a film made of a resin and coating the wire 24. Presence of the coated portion 27 provides the resin-coated wire 20 with high slidability, as well as reduces loss of the drug solution DS due to permeation of the drug solution DS into the wire 24 (in a space(s) between the fine wires 240 and/or between the strands 242).

The coated portion 27 has a thickness of e.g., about 0.1 mm to 0.2 mm. The resin-coated wire 20 thus has an outer shape approximately identical to the outer shape of the wire 24. In other words, the outer shape of the most distal end portion P1 of the resin-coated wire 20 is an approximate hemisphere shape projecting toward the distal end, as with the outer shape of the welded portion 23 of the wire 24. Additionally, the outer shape of the intermediate portion P2 adjacent to a proximal end side of the most distal end portion P1 is a taper shape with an outer diameter reduced toward the distal end, as with the outer shape of the tapered portion 22 of the wire 24. Furthermore, the outer shape of the proximal end portion P3 adjacent to a proximal end side of the intermediate portion P2 is a straight shape with an approximately constant outer diameter, as with the outer shape of the straight portion 21 of the wire 24.

The coated portion 27 has a distal end coated portion 25 and a proximal end coated portion 26. The distal end coated portion 25 coats the welded portion 23 and the tapered portion 22 of the wire 24. That is, the distal end coated portion 25 forms the distal end portion P4 of the resin-coated wire 20. The proximal end coated portion 26 coats the straight portion 21 of the wire 24. That is, the proximal end coated portion 26 forms the proximal end portion P3 of the resin-coated wire 20.

The distal end coated portion 25 and the proximal end coated portion 26 are formed of different types of resin materials. More particularly, a resin material for forming the distal end coated portion 25 has a higher affinity for water than a resin material for forming the proximal end coated portion 26. Examples of a combination of two resin materials satisfying such a condition include a combination where a resin material for forming the proximal end coated portion 26 is ETFE, PTFE, or PFA, and where a resin material for forming the distal end coated portion 25 is acrylic urethane, polyethylene, or polyamide. Note that a drug solution to be employed in the drug solution injection device 100 in the embodiment (hereinafter referred to as "drug solution of interest") is prepared by dissolving a drug into a physiological saline, and thus contains a large amount of water. Therefore, a resin material for forming the distal end coated portion 25 is more compatible with a drug solution of interest than a resin material for forming the proximal end coated portion 26. Affinity for water in a resin material can be represented by a contact angle, and a smaller contact angle indicates higher compatibility with water. A resin material for forming the distal end coated portion 25 is an exemplary first resin material, and a resin material for forming the proximal end coated portion 26 is an exemplary second resin material.

Moreover, in the embodiment, as depicted in FIG. 3 and FIG. 4, a resin material for forming the distal end coated portion 25 (e.g., acrylic urethane, polyethylene, or polyamide) permeates a distal end part of the straight portion 21 of the wire 24 (hereinafter referred to as "distal straight portion 28"). In other words, in the distal straight portion 28, a resin material for forming the distal end coated portion 25 is present in a space 29 between the fine wires 240 and/or between the strands 242, which are included in the wire 24.

In the resin-coated wire 20 in the embodiment, the distal end portion P4 includes the welded portion 23 to which each of the fine wires 240 and/or each of the strand 242 included in the wire 24 are joined. Additionally, in the distal end portion P4, a resin material forming the distal end coated portion 25 permeates between the fine wires 240 and/or between the strands 242 which form the wire 24, thereby adhering the fine wire 240 to one another and the strands 242 to one another. The distal end portion P4 (a part including the welded portion 23 and the tapered portion 22 of the wire 24) of the resin-coated wire 20 thus has higher rigidity than that of the proximal end portion P3 (a part including the straight portion 21 of the wire 24).

### A-3. Production Method of Resin-coated Wire 20:

An exemplary production method of the resin-coated wire 20 included in the drug solution injection device 100 in the embodiment is as described below. At first, a rope wire fully coated by the proximal end coated portion 26 (a material for forming the wire 24) is prepared. In the rope wire, a part to be finally the tapered portion 22 of the wire 24 is preliminarily processed into a taper shape.

Next, in the rope wire, a piece of the proximal end coated portion 26 on a part to be finally the tapered portion 22 and the welded portion 23 of the wire 24 is peeled off. This causes the proximal end coated portion 26 to coat only a part to be the straight portion 21 of the wire 24 and to expose a part to be the tapered portion 22 and the welded portion 23 of the wire 24.

Subsequently, the most distal end portion of the rope wire is subject to welding from an axis direction by, e.g., YAG laser welding, thereby forming the welded portion 23. This causes formation of the wire 24, which has the straight portion 21, the tapered portion 22, and the welded portion 23.

Then, a material for forming the distal end coated portion 25 (adhesive) is applied to the part where the proximal end coated portion 26 is peeled off in the rope wire. This causes formation of the distal end coated portion 25 coating the tapered portion 22 and the welded portion 23 of the wire 24. Additionally, in the straight portion 21 of the wire 24, a material for forming the distal end coated portion 25 permeates the distal straight portion 28, and adheres the fine wires 240 to one another and/or the strand 242 to one another, all of which are included in the wire 24. This process allows production of the resin-coated wire 20 according to the embodiment.

### A-4. Effects of the Embodiment:

As has been described above, the drug solution injection device 100 of the present embodiment includes the resin-coated wire 20, which is capable of moving forward inside the drug solution-containing lumen L of the needle catheter 10 containing the drug solution DS, and thereby pushing out the drug solution DS from the distal end of the needle catheter 10. The resin-coated wire 20 includes the wire 24, and the coated portion 27 made of resin coating the wire. The coated portion 27 has the distal end coated portion 25 and the proximal end coated portion 26. The distal end coated portion 25 coats the distal end portion of the wire 24 (the welded portion 23 and the tapered portion 22). The proximal end coated portion 26 coats a part (the straight portion 21) adjacent to a proximal end side of the distal end portion (the welded portion 23 and the tapered portion 22) in the wire 24. A resin material for forming the distal end coated portion 25 has a higher affinity for water than a resin material for forming the proximal end coated portion 26.

Accordingly, in the resin-coated wire 20 according to the embodiment, a resin material for forming the distal end coated portion 25 has a higher affinity for water than a resin material for forming the proximal end coated portion 26. In other words, a bubble in the water (in the drug solution DS) is easily attached so as to spread on a surface of the proximal end coated portion 26, while the bubble poorly spreads and insufficiently attaches onto a surface of the distal end coated portion 25. Hence, even when a bubble is mixed in the drug solution DS contained in the drug solution-containing lumen L of the needle catheter 10, the bubble can be prevented from flowing to the distal end by moving the resin-coated wire 20 forward and causing the bubble to move from a position of the distal end coated portion 25 to a position of the proximal end coated portion 26 and attach to the proximal end coated portion 26. Accordingly, the resin-coated wire 20 in the embodiment can reduce a risk of administering a bubble together with the drug solution DS into an affected area, in pushing out the drug solution DS from a distal end of the needle catheter 10 using the resin-coated wire 20.

In the resin-coated wire 20 according to the embodiment, the wire 24 is a rope wire formed by spirally winding a plurality of the fine wires 240. The resin-coated wire 20 according to the embodiment thus can increase flexibility of the resin-coated wire 20, easily move the resin-coated wire 20 forward inside the drug solution-containing lumen L of the needle catheter 10 even when the needle catheter 10 is bent, and maneuverability of the resin-coated wire 20, accuracy of drug solution injection by the resin-coated wire 20, etc. is improved.

In the resin-coated wire 20 according to the embodiment, each of the plurality of the fine wires 240 forming the wire 24 is a stranded wire formed by spirally winding a plurality of the strands 242. The resin-coated wire 20 in the embodiment can thus effectively increase flexibility of the resin-coated wire 20, easily move the resin-coated wire 20 forward inside the drug solution-containing lumen L of the needle catheter 10 even at a bend of the needle catheter 10, and maneuverability of the resin-coated wire 20, accuracy of drug solution injection by the resin-coated wire 20, etc. is effectively improved.

In the resin-coated wire 20 according to the embodiment, a resin material for forming the distal end coated portion 25 permeates the distal straight portion 28, which is a distal end part of the straight portion 21 of the wire 24. The resin-coated wire 20 in the embodiment can thus effectively reduce loss of the drug solution DS due to e.g., infiltration of the drug solution DS from a boundary between the proximal end coated portion 26 and the distal end coated portion 25 to an inside of the wire 24. The resin-coated wire 20 can also avoid a sharp change of rigidity of the wire 24 (in turn, rigidity of the resin-coated wire 20) from the distal end to the proximal end of the wire 24, and maneuverability of the resin-coated wire 20, accuracy of drug solution injection by the resin-coated wire 20, etc. is improved.

In the resin-coated wire 20 according to the embodiment, the most distal end portion P1 in the resin-coated wire 20 has an approximate hemisphere shape forming a projection toward the distal end. The resin-coated wire 20 according to the embodiment can thus easily move the resin-coated wire 20 forward inside the drug solution-containing lumen L of the needle catheter 10 even when the needle catheter 10 is bent, and maneuverability of the resin-coated wire 20, accuracy of drug solution injection by the resin-coated wire 20, etc. is effectively improved.

The resin-coated wire 20 according to the embodiment includes the most distal end portion P1, the intermediate portion P2 adjacent to a proximal end side of the most distal end portion P1 and having a taper shape with an outer diameter reduced toward the distal end, and the proximal end portion P3 adjacent to a proximal end side of the intermediate portion P2 and having an approximately constant outer diameter. Owing to having the tapered intermediate portion P2, the resin-coated wire 20 according to the embodiment can thus easily move the resin-coated wire 20 forward inside the drug solution-containing lumen L of the needle catheter 10 even when the needle catheter 10 is bent, and maneuverability of the resin-coated wire 20, accuracy of drug solution injection by the resin-coated wire 20, etc. is effectively improved.

In the resin-coated wire 20 according to the embodiment, a distal end portion P4 (a part formed of the welded portion 23 and the tapered portion 22 of the wire 24) has higher rigidity than that of the proximal end portion P3 (a part formed of the straight portion 21 of the wire 24). The resin-coated wire 20 according to the embodiment can reduce a risk of deformation or backward movement of a vicinity of the distal end of the resin-coated wire 20 due to a pressure from the drug solution DS, in moving the resin-coated wire 20 forward inside the drug solution-containing lumen L of the needle catheter 10, and maneuverability of the resin-coated wire 20, accuracy of drug solution injection by the resin-coated wire 20, etc. is effectively improved.

The drug solution injection device 100 in the embodiment includes the resin-coated wire 20 and the needle catheter 10 in the aforementioned configuration. The drug solution injection device 100 according to the embodiment thus can improve maneuverability, accuracy of drug solution injection, etc. of the drug solution injection device 100.

### B. Modification:

The technology disclosed herein is not limited to the aforementioned embodiment, and can be modified into various forms within a range not departing from the scope of the invention. For example, the following modifications are also available.

The configuration of the drug solution injection device 100 in the aforementioned embodiment is solely an example, and can be variously modified. For example, in the aforementioned embodiment, the wire 24 includes the welded portion 23, the tapered portion 22, and the straight portion 21, but the straight portion 21 has another portion close to the proximal end. The wire 24 may not have the welded portion 23 and/or the tapered portion 22.

In the aforementioned embodiment, each of a plurality of the fine wires 240 forming the wire 24 is a stranded wire formed by spirally winding a plurality of the strands 242, but each of the plurality of the fine wires 240 forming the wire 24 may be formed of a single strand. Additionally, in the aforementioned embodiment, the wire 24 is a rope wire formed by spirally winding a plurality of the fine wires 240, but the wire 24 may be formed of a single strand.

In the aforementioned embodiment, the distal end coated portion 25 in the coated portion 27 coats the welded portion 23 and the tapered portion 22 of the wire 24, and the proximal end coated portion 26 coats the straight portion 21 of the wire 24, but the configuration of the coated portion 27 is not limited thereto. For example, the distal end coated portion 25 may coat the welded portion 23 and a part close to the distal end in the tapered portion 22, and the proximal end coated portion 26 may coat a part close to the proximal end in the tapered portion 22 and the straight portion 21. Alternatively, the distal end coated portion 25 may coat the welded portion 23, the tapered portion 22, and a part close to the distal end in the straight portion 21, and the proximal end coated portion 26 may coat a part close to the proximal end in the straight portion 21.

A material for forming each member included in the drug solution injection device 100 according to the embodiment is solely an example, and can be variously modified. A method of producing the resin-coated wire 20, which is included in the drug solution injection device 100 according to the embodiment, is also solely an example, and can be variously modified.

### DESCRIPTION OF REFERENCE NUMERALS

10: needle catheter
12: catheter shaft
14: needle portion
16: inner cavity
18: inner cavity
20: resin-coated wire
21: straight portion
22: tapered portion
23: welded portion
24: wire
25: distal end coated portion
26: proximal end coated portion
27: coated portion
28: distal end straight portion
29: space
30: controller
100: drug solution injection device
240: fine wire
242: strand
AX: center axis
DS: drug solution
L: drug solution-containing lumen
P1: the most distal end portion
P2: intermediate portion
P3: proximal end portion
P4: distal end portion

## Claims

1. A resin-coated wire capable of moving forward inside a drug solution-containing lumen of a catheter containing a drug solution, and thereby pushing out the drug solution from a distal end of the catheter, the resin-coated wire comprising:
a wire; and
a coated portion made of resin coating the wire,
wherein the coated portion includes:
a distal end coated portion formed of a first resin material and coating a first part being a distal end portion of the wire; and
a proximal end coated portion formed of a second resin material and coating a second part adjacent to a proximal end side of the first part of the wire, and
wherein the first resin material has a higher affinity for water than the second resin material.

2. The resin-coated wire according to claim 1, wherein the wire is a rope wire formed by spirally winding a plurality of fine wires.

3. The resin-coated wire according to claim 2, wherein each of the plurality of fine wires is a stranded wire formed by spirally winding a plurality of strands.

4. The resin-coated wire according to claim 2 or claim 3, wherein the first resin material permeates a distal end part of the second part of the wire.

5. The resin-coated wire according to any one of claim 1 to claim 4, wherein a most distal end portion of the resin-coated wire has an approximate hemisphere shape forming a projection toward a distal end.

6. The resin-coated wire according to any one of claim 1 to claim 5, wherein the resin-coated wire includes:
a most distal end portion;
an intermediate portion adjacent to a proximal end side of the most distal end portion and having a taper shape with an outer diameter reduced toward the distal end; and
a proximal end portion adjacent to a proximal end side of the intermediate portion and having an approximately constant outer diameter.

7. The resin-coated wire according to any one of claim 1 to claim 6, wherein a part formed of the first part of the wire has higher rigidity than that of a part formed of the second part of the wire.

8. A drug solution injection device comprising:
the resin-coated wire according to any one of claim 1 to claim 7; and
the catheter.
